# EUROPEAN PATENT APPLICATION

(11) **EP 1 736 542 A1**
(43) Date of publication of application: **27.12.2006**
(21) Application number: 05726649.6
(22) Date of filing: 17.03.2005
(51) Int. Cl.: C12N 15/09, A01N 1/00, G01N 33/50

(54) **SOLVENT FOR DISSOLVING NUCLEIC ACID, NUCLEIC ACID-CONTAINING SOLUTION AND METHOD OF PRESERVING NUCLEIC ACID**

(30) Priority: 23.03.2004 JP 2004084702; 26.07.2004 JP 2004217552
(71) Applicant: Ohno, Hiroyuki, Tokyo 134-0092 (JP); Nisshinbo Industries, Inc., Chuo-ku, Tokyo 103-8650 (JP)
(72) Inventor: OHNO, Hiroyuki;, Edogawa-ku, Tokyo 1340092; (JP); FUKAYA, Yukinobu, 1870011 (JP); MASUDA, Gen, c/o R & D Center, NISSHINBO IND., INC, Chiba-shi, Chiba 267-0056 (JP)
(74) Representative: Stuart, Ian Alexander
(86) International application number: PCT/JP2005/004773
(87) International publication number: WO 2005/090563

(57) **Abstract**

As a solvent for dissolving a nucleic acid such as DNA or RNA, a solvent comprising an ionic liquid having, for example, ammonium cation, imidazolium cation orpyridinium cation is used and the nucleic acid is dissolved therein and preserved. Thus, a nucleic acid such as DNA or RNA can be easily dissolved and preserved over a long period of time.

## Description

### TECHNICAL FIELD

The present invention relates to a solvent for dissolving nucleic acid, a nucleic acid-containing solution, and a method of preserving nucleic acid. More specifically, the invention relates to a solvent for dissolving nucleic acids such as DNA and RNA which is composed of an ionic liquid, and relates also to a nucleic acid-containing solution and a method of preserving nucleic acid which use such solvents.

### BACKGROUND ART

DNA and RNA are genetic substances which govern heredity. Efforts are being made in many aspects to achieve functional improvements in biological species at the level of the gene by using techniques from biotechnology that, for example, decode genomic information and rearrange gene sequences.
Nor are applications of DNA and RNA limited only to the field of biotechnology. Rapid progress is being made on the use of novel diagnostic and therapeutic techniques in the field of health care, such as genetic testing, gene therapy, and elucidation of the etiology of diseases, by employing genetic information on DNA and RNA.

These nucleic acids, which are biopolymers present in all living things, are also noteworthy as materials in a number of respects. For example, they are very abundant in nature and are biodegradable. Moreover, DNA and RNA are materials which, through chemical techniques, can be imparted with even higher functionality. Hence, research is also being done on the use of DNA and RNA as industrial materials.

Yet, DNA is a chain-like macromolecule composed of deoxyribonucleotide monomer units, each of which includes one molecule of a nitrogen-containing heterocyclic base which is a derivative of purine or pyrimidine (i.e., one nucleic acid base molecule), one deoxyribose molecule, and one phosphoric acid molecule. Similarly, RNA is a covalent bonded chain of ribonucleotide monomer units, each containing one molecule of a nucleic acid base, one molecule of ribose, and one molecule of phosphoric acid.
Given that DNA and RNA are macromolecular compounds in which the hydrophilic phosphoric acid and sugar form the main skeleton, they are very highly hydrophilic substances.

Because DNA and RNA are so highly hydrophilic, they have until now been handled in systems which use water as the solvent.
Although water is a good solvent for dissolving nucleic acids, the temperature range of about 0 to 100°C in which it can be used is narrow. Moreover, the use of water as the solvent makes it impossible to carry out reactions using reagents and the like which cannot be handled in water. These and other drawbacks have limited the conditions for the industrial use of nucleic acids. Hence, there exists a need for solvents which have a broad usable temperature range and in which reactions that proceed with difficulty or not at all in water can be carried out.

Also, DNA and RNA are known to readily incur hydrolysis in water under the action of, respectively, deoxyribonuclease (DNase) and ribonuclease (RNase). When storing and preserving DNA and RNA, it is necessary to dissolve them in water from which DNase and RNase have been removed.
However, because water is a solvent that readily evaporates, designing a container for such preservation is tricky. Furthermore, if DNase or RNase is present in a nucleic acid specimen that has been directly extracted from a living organism, or if nuclease present in the saliva or adhering to the skin of an organism enters the system externally, the DNA or RNA is readily degraded under the activity of the enzyme that is present within or has inadvertently entered the system, making the long-term preservation of DNA or RNA using water as the solvent impossible.

A method is also known for enabling the long-term preservation of DNA and RNA by using reagents which deactivate DNase and RNase. However, the reagents used for this purpose are sometimes cytotoxic or carcinogenic. Moreover, because there is also a possibility that such reagents may chemically modify the nucleic acid bases, this approach is not simple or convenient as a method of preservation.
It may be possible to preserve the nucleic acid in the absence of water by freeze-drying the nucleic acid-containing specimen. However, such a method cannot be employed in places without freeze-drying equipment. Moreover, after drying, the specimen must be stored at 0°C or below. Hence, this method of preservation also lacks simplicity and convenience.

It has been reported that, in an aqueous solution containing a high concentration of an inorganic salt, the higher order structure of DNase and RNase is disrupted, resulting in degradation (Non-Patent Document 1: P.J. von Hippel: J. Biol. Chem. 10, 3913 (1965).
It would thus seem that using this technique to dissolve DNA and RNA in an aqueous solution having a high salt concentration should be a convenient method for long-term preservation.
However, here too, water is used as the solvent. As a result, not only is it necessary to find some way to prevent the water from evaporating, complicated operations are required for desalting the DNA and RNA following preservation. Hence, this approach also lacks suitability as a method for long-term preservation.
Accordingly, there exists a desire for the establishment of a technique capable of easily and conveniently preserving DNA and RNA for an extended period of time.

Non-Patent Document 1: P.J. von Hippel: J. Biol. Chem. 10, 3913 (1965)

### DISCLOSURE OF THE INVENTION

### Problems to be Solved by the Invention

It is therefore an object of the invention to provide a solvent for dissolving nucleic acid which can easily dissolve nucleic acids such as DNA and RNA, and can preserve those nucleic acids for a long period of time. Further objects of the invention are to provide a nucleic acid-containing solution and a method of preserving nucleic acid which use such a solvent.

### Means for Solving the Problems

As a result of extensive investigations, we have discovered that salts which are liquid at room temperature, that is, ionic liquids, are able to easily dissolve DNA and RNA. We have also found that DNA and RNA can be preserved for a long period of time in a dissolved state within an ionic liquid.
Accordingly, the invention provides the following.
[1] A solvent for dissolving nucleic acid, characterized by comprising an ionic liquid which can dissolve nucleic acid.
[2] The solvent for dissolving nucleic acid of [1] which is characterized in that the ionic liquid is composed of at least one cation selected from among ammonium cations, imidazolium cations and pyridinium cations.
[3] The solvent for dissolving nucleic acid of [1] or [2] which is characterized in that the ionic liquid is composed of an anion which is a halide ion or a carboxylic acid ion having a total of 1 to 3 carbons.
[4] The solvent for dissolving nucleic acid of [1] which is characterized in that the ionic liquid is a neutralized ionic liquid.
[5] The solvent for dissolving nucleic acid of any one of [1] to [4] which is characterized by being adapted to preserve nucleic acid or to react nucleic acid.
[6] A nucleic acid-containing solution, characterized by comprising a nucleic acid dissolved in an ionic liquid.
[7] A method of preserving nucleic acid, characterized by preserving nucleic acid in a dissolved state within an ionic liquid.

### Effects of the Invention

Because the inventive solvent for dissolving nucleic acid is composed of an ionic liquid which can dissolve nucleic acid, the solvent is able not only to easily dissolve DNA and RNA, it is also able to preserve for a long time the DNA and RNA in a dissolved state within the ionic liquid.
That is, because ionic liquids are liquids that have a high ion density and a high polarity equivalent to that of water, they are capable of dissolving DNA and RNA molecules which have numerous hydrophilic groups.
Also, perhaps because nucleases are deactivated within the ionic liquid on account of the very high ionic strength of the liquid, DNA and RNA can be stably preserved for a long time by being dissolved in the ionic liquid. In addition, given that ionic liquids have a vapor pressure that is either very low or nonexistent, they do not evaporate even at elevated temperatures and reduced pressures. Hence, the basic performance of an ionic liquid can be sustained over a long period of time without special modifications to the container used during preservation. This is yet another reason why ionic liquids are best suited for the long-term preservation of nucleic acids.
Furthermore, because ionic liquids are substances which exhibit a liquid state over a broad temperature range, when an ionic liquid is used as a reaction solvent for nucleic acid, unlike the water used for this purpose until now, the reaction can be carried out over a broad temperature range. In addition, the use of ionic liquids in this way makes it possible to carry out reactions that employ reagents which cannot be handled in water.

### BRIEF DESCRIPTION OF THE DIAGRAMS

FIG. 1 shows the visible-ultraviolet absorption spectra obtained in Examples 9 and 10.
FIG. 2 shows the visible-ultraviolet absorption spectra obtained in Examples 11 and 12.
FIG. 3 shows the visible-ultraviolet absorption spectrum obtained in Example 13.
FIG. 4 shows the CD spectrum obtained in Example 13.
FIG. 5 shows the visible-ultraviolet spectrum obtained in Example 14.
FIG. 6 shows the CD spectrum obtained in Example 14.

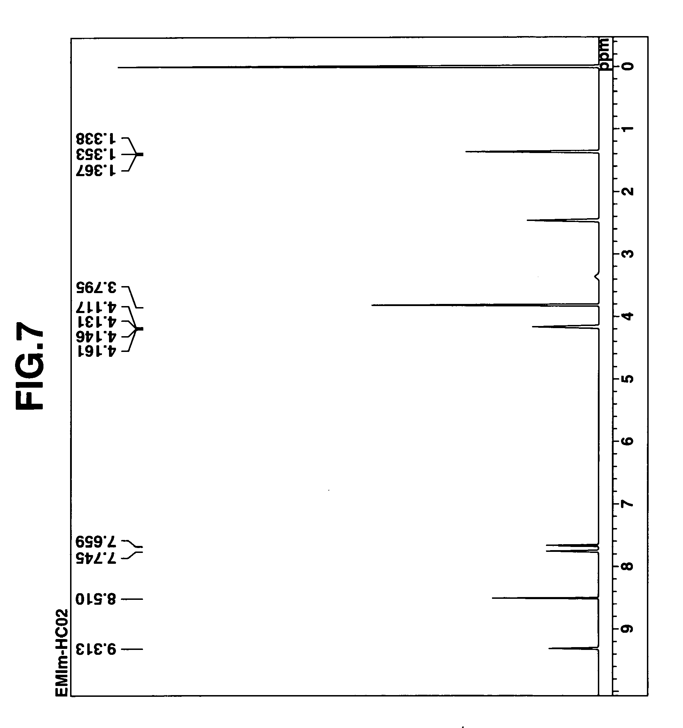

FIG. 7 shows the NMR chart for the ionic liquid EMIm-HCOO obtained in Synthesis Example 8.

### BEST MODE FOR CARRYING OUT THE INVENTION

The invention is described more fully below.
The inventive solvent for dissolving nucleic acid is composed of an ionic liquid.
The ionic liquid is not subject to any particular limitation, provided it is capable of dissolving nucleic acid (nucleic acid-dissolving). In the practice of the invention, it is also possible to use a neutralized ionic liquid.
As used herein, "neutralized ionic liquid" refers to an ionic liquid composed of a salt obtainable by an acid-base neutralization reaction (see Ion-sei ekitai--Kaihatsu no saizensen to mirai-- [Ionic liquids: The frontiers of current research and the future], published by CMC Shuppan (2003), pp. 19-21). Such ionic liquids are characterized by having cations obtained by proton addition.

For nucleic acid to have an outstanding solubility in the ionic liquid, it is preferable that the cation making up the ionic liquid be at least one selected from among ammonium cations, imidazolium cations and pyridinium cations. Of these, imidazolium cations are especially preferred because they have an excellent ability to dissolve nucleic acid and are capable of preparing high-concentration nucleic acid-containing solutions.
In the practice of the invention, "capable of dissolving nucleic acid (nucleic acid-dissolving)" means that a cloudy liquid obtained by adding not more than about 10 wt%, preferably not more than about 5.0 wt%, of nucleic acid to a solvent, when subsequently rendered into a clear and homogeneous solution by dissolving the nucleic acid under the application of heat, for example, remains in a precipitation-free state even on cooling to room temperature (about 20°C).

Illustrative, non-limiting, examples of the imidazolium cation include monoalkylimidazolium cations, dialkylimidazolium cations and trialkylimidazolium cations. Specific examples include the 1-ethyl-3-methylimidazolium ion,
the 1-butyl-3-methylimidazolium ion,
the 1-propyl-3-methylimidazolium ion,
the 1-(1-, 2- or 3-hydroxypropyl)-3-methylimidazolium ion,
the 1,2,3-trimethylimidazolium ion,
the 1,2-dimethyl-3-ethylimidazolium ion,
the 1,2-dimethyl-3-propylimidazolium ion,
the 1-butyl-2,3-dimethylimidazolium ion,
the imidazolium ion, the 1-methylimidazolium ion,
the 1-ethylimidazollum ion, and the 1,2-dimethylimidazolium ion.
Illustrative, non-limiting, examples of the pyridinium cation include the N-propylpyridinium ion,
the N-butylpyridinium ion, the 1-butyl-4-methylpyridinium ion,
the 1-butyl-2,4-dimethylpyridinium ion,
the 2-methylpyridinium ion, the 3-methylpyridinium ion, and
the 4-methylpyridinium ion.

Ammonium cations that may be used as the cation component are not subject to any particular limitation, although aliphatic or alicyclic ammonium ions are preferred.
Illustrative, non-limiting, examples of such aliphatic and alicyclic ammonium ions include primary to quaternary alkyl ammonium ions such as the diethylammonium ion, the triethylammonium ion, the methoxyethylammonium ion, the diethyl(2-methoxyethyl)ammonium ion, the trimethylpropylammonium ion, the trimethylhexylammonium ion, the tetrapentylammonium ion, and the diethylmethyl(2-methoxyethyl)ammonium ion; and the N-methylpyrrolidinium, ion and
the N-butyl-N-methylpyrrolidinium ion.

Examples of anions that may be used as the anion making up the ionic liquid include BF₄⁻, PF₆⁻, AsF₆⁻, SbF₆⁻, AlCl₄⁻, HSO₄⁻, ClO₄⁻, CH₃SO₃⁻, CF₃SO₃⁻, CF₃CO₂⁻, (CF₃SO₂)₂N⁻, C₂H₅CO₂⁻, CH₃CO₂⁻, HCO₂⁻, Cl⁻, Br⁻ and I⁻. To increase the solubility of the nucleic acid, a halide ion or a carboxylic acid ion having a total of 1 to 3 carbons is preferred. Of these, Cl⁻, Br⁻ and HCO₂⁻ are especially preferred.

The ionic liquid is preferably one which has been vacuum dehydrated under heating. The water content is preferably not more than about 1.0 wt%, and more preferably not more than about 0.5 wt%. The heating temperature and degree of vacuum during dehydration may be selected as appropriate for the type of ionic liquid. The water content is a measured value obtained with a Karl Fischer moisture titrator.

The nucleic acid-containing solution according to the invention is a solution obtained by dissolving a nucleic acid in the solvent for dissolving nucleic acid composed of the above-described ionic liquid.
The nucleic acid content in the ionic liquid cannot be strictly specified due to fluctuations in solubility depending on the type of ionic liquid used, but is generally not more than 10 wt%, and preferably in a range of about 1.0 to about 5.0 wt%.

No particular limitation is imposed on the method of dissolving nucleic acid in the ionic liquid, although use may be made of a method which involves adding a given amount of nucleic acid to the ionic liquid, then heating to about 70 to 120°C so as to dissolve the nucleic acid. Whether the nucleic acid has dissolved in the ionic liquid can be ascertained by checking if the cloudy solution obtained by the addition of nucleic acid turns uniformly clear after heating.
The nucleic acid-containing solution prepared using the inventive solvent for dissolving nucleic acid, when heated to dissolve the nucleic acid and obtain a uniformly clear state, then cooled once again to about 10 to 25°C, does not undergo nucleic acid precipitation and exhibit clouding.

The solvent for dissolving nucleic acid and the nucleic acid-containing solution described above may be used in all applications requiring the presence of dissolved nucleic acids such as DNA or RNA.
Specific examples include using the solvent for dissolving nucleic acid to preserve nucleic acid, and storing the nucleic acid in the form of a nucleic acid solution, thereby preserving the nucleic acid in an environment that deactivates nucleases and thus enabling the very convenient long-term and stable storage of nucleic acid. In such a case, although the storage temperature is not subject to any particular limitation, because storage at a general ambient temperature of about 0 to 40°C is possible, long-term storage at room temperature can be carried out in the absence of a special environment.
Moreover, by using the solvent for dissolving nucleic acid in a nucleic acid reaction and carrying out, for example, a DNA or RNA chemical modification reaction in this solvent, reactions which use reagents that cannot be handled in the water hitherto used as the solvent and reactions which have difficulty proceeding in water can be carried out without being restricted to a temperature range of 0 to 100°C.

### EXAMPLES

Synthesis examples and examples of the invention are given below by way of illustration and not by way of limitation. Structural confirmation of the ionic liquid in these examples was carried out using a ¹H-NMR spectrometer (Alpha-500, manufactured by JEOL Ltd.). In Synthesis Examples 6 to 8, the absence of halide in the product was confirmed from the fact that the clouding of the solution that accompanies the formation of silver halide did not occur when a 0.1 M solution of silver nitrate in water (available from Kanto Chemical Co., Inc.) was added. Also, the DNA or RNA was regarded as having dissolved in the ionic liquid when the liquid changed from a cloudy state to a uniformly clear solution.

### Synthesis Example 1

Ten grams of N-methylimidazole (Aldrich Chemical Co., Inc.) and 12 g of 3-chloro-1-propanol (Tokyo Chemical Industry Co., Ltd.) were mixed and the mixture was stirred for seven days in a nitrogen atmosphere under refluxing at 60°C, following which the reaction product was added dropwise to 300 ml of diethyl ether (Kanto Chemical) and the liquid that precipitated out was collected. The precipitate was dissolved in 10 ml of methanol (Wako Pure Chemical Industries, Ltd.), the resulting solution was added dropwise to 300 ml of diethyl ether, and the liquid that precipitated out was collected. The obtained liquid was dissolved in 300 ml of ethanol (Wako Pure Chemical Industries), 20 g of activated carbon powder (Wako Pure Chemical Industries) was added, and the mixture was stirred for 24 hours. The activated carbon powder was then removed by filtration and the solvent was driven off by distillation under a reduced pressure, giving the ionic liquid HO(CH₂)₃MIN-Cl.

### Synthesis Example 2

Five grams of N-methylimidazole and 8.5 g of 3-bromo-1-propanol (Aldrich Chemical) were mixed and the mixture was stirred for three days in a nitrogen atmosphere under refluxing at 60°C, following which the reaction product was added dropwise to 250 ml of diethyl ether and the liquid that precipitated out was collected. The precipitate was dissolved in 10 ml of methanol, the resulting solution was added dropwise to 300 ml of diethyl ether, and the liquid that precipitated out was collected. The obtained liquid was dissolved in 300 ml of methanol, 20 g of activated carbon powder was added, and the mixture was stirred for 24 hours. The activated carbon powder was then removed by filtration and the solvent was driven off by distillation under a reduced pressure, giving the ionic liquid HO(CH₂)₃MIN-Br.

### Synthesis Example 3

Ten grams of N-methylimidazole and 50 g of 1-chloropropane (Tokyo Chemical Industry) were mixed and the mixture was stirred for seven days in a nitrogen atmosphere under refluxing at 45°C, following which the unreacted 1-chloropropane was removed by distillation, the reaction product was added dropwise to 300 ml of diethyl ether, and the liquid that precipitated out was collected. The precipitate was dissolved in 10 ml of methanol, the resulting solution was added dropwise to 300 ml of diethyl ether, and the liquid that precipitated out was collected. The obtained liquid was dissolved in 300 ml of acetonitrile (Wako Pure Chemical Industries), 20 g of activated carbon powder was added, and the mixture was stirred for 24 hours. The activated carbon powder was then removed by filtration and the solvent was driven off by distillation under a reduced pressure, giving the ionic liquid PMIN-Cl.

### Synthesis Example 4

Ten grams of N-methylimidazole and 50 g of 1-bromopropane (Kanto Chemical) were mixed and the mixture was stirred for four days in a nitrogen atmosphere under refluxing at 50°C, following which the reaction product was added dropwise to 300 ml of diethyl ether and the liquid that precipitated out was collected. The precipitate was dissolved in 10 ml of methanol, the resulting solution was added dropwise to 300 ml of diethyl ether, and the liquid that precipitated out was collected. The obtained liquid was dissolved in 300 ml of acetonitrile, 20 g of activated carbon powder was added, and the mixture was stirred for 24 hours. The activated carbon powder was then removed by filtration and the solvent was driven off by distillation under a reduced pressure, giving the ionic liquid PMIN-Br.

### Synthesis Example 5

Ten grams of N-methylimidazole (Aldrich Chemical) and 20 ml of bromoethane (Kanto Chemical) were mixed and the mixture was reacted for two days in a nitrogen atmosphere at 0°C. The reaction product was then added dropwise to 300 ml of diethyl ether (Kanto Chemical), and the white solid that precipitated out was collected. The precipitate was added to 300 ml of diethyl ether and stirred for two hours, following which the precipitated white solid was recovered. The remaining solvent was driven off in vacuo, giving EMIm-Br.

### Synthesis Example 6

A solution prepared by dissolving 2 grams of the EMIm-Br obtained in Synthesis Example 5 in 60 ml of deionized water was passed through a column packed with 150 ml of the anion exchange resin Amberlite IRA-400 (OH) (available from Sperco), thereby giving an aqueous solution of EMIm-OH.

### Synthesis Example 7

Sixty milliliters of the aqueous solution of EMIm-OH obtained in Synthesis Example 6 and 0.8 g of propionic acid (Kanto Chemical) were mixed and the mixture was stirred on an ice bath for 24 hours, following which the water was driven off in vacuo, giving the reaction product. The reaction product was added dropwise to 300 ml of diethyl ether (Kanto Chemical), and the liquid that precipitated out was collected. The precipitate was dissolved in 5 ml of methanol (Wako Pure Chemical Industries), and the solution was added dropwise to 300 ml of diethyl ether, following which the liquid that precipitated out was collected. Residual solvent was driven from the collected precipitate under reduced pressure, yielding the ionic liquid EMIm-C₂COO.

### Synthesis Example 8

Sixty milliliters of the aqueous solution of EMIm-OH obtained in Synthesis Example 6 was mixed with 0.5 g of formic acid (Wako Pure Chemical Industries) and the mixture was stirred for 24 hours on an ice bath, following which the water was driven off in vacuo, giving the reaction product. The reaction product was added dropwise to 300 ml of diethyl ether (Kanto Chemical), and the liquid that precipitated out was collected. The precipitate was dissolved in 5 ml of methanol (Wako Pure Chemical Industries), and the solution was added dropwise to 300 ml of diethyl ether, following which the liquid that precipitated out was collected. Residual solvent was driven from the collected precipitate under reduced pressure, yielding the ionic liquid EMIm-HCOO.

### Synthesis Example 9

Five grams (61 mmol) of N-methylimidazole (Acros Organics) was mixed with 30.5 ml of an aqueous solution of hydrochloric acid prepared by diluting hydrochloric acid (Wako Pure Chemical Industries) to 2 mol/L with distilled water, and the mixture was stirred for 24 hours on an ice bath. The mixture was then freeze-dried and residual water was driven off under reduced pressure, giving the ionic liquid MIm-Cl.

### Synthesis Example 10

Five grams (61 mmol) of N-methylimidazole (Acros Organics) was mixed with 2.3 ml (61 mmol) of formic acid (Wako Pure Chemical Industries), and the mixture was stirred for 24 hours on an ice bath. The water was then driven off under reduced pressure, giving the ionic liquid MIm-HCOO.

### Example 1

One gram of the ionic liquid HO(CH₂)₃MIN-Cl prepared in Synthesis Example 1 was vacuum dehydrated under heating at 110°C for 24 hours, then mixed with 10 mg of DNA (Daiwa Fine Chemical Co., Ltd.) and sealed in a nitrogen atmosphere. Next, while applying heat, the temperature at which the DNA dissolved was recorded. As a result, the temperature at which 10 mg of DNA completely dissolves in 1 g of the ionic liquid HO(CH₂)₃MIN-Cl was found to be 70°C.

### Example 2

One gram of the ionic liquid HO(CH₂)₃MIN-Cl prepared in Synthesis Example 1 was vacuum dehydrated under heating at 110°C for 24 hours, then mixed with 10 mg of RNA (Tokyo Kasei Kogyo) and sealed in a nitrogen atmosphere. Next, while applying heat, the temperature at which the RNA dissolved was recorded. As a result, the temperature at which 10 mg of RNA completely dissolves in 1 g of the ionic liquid HO(CH₂)₃MIN-Cl was found to be 60°C.

### Example 3

One gram of the ionic liquid HO(CH₂)₃MIN-Br prepared in Synthesis Example 2 was vacuum dehydrated under heating at 110°C for 24 hours, then mixed with 10 mg of DNA and sealed in a nitrogen atmosphere. Next, while applying heat, the temperature at which the DNA dissolved was recorded. As a result, the temperature at which 10 mg of DNA completely dissolves in 1 g of the ionic liquid HO(CH₂)₃MIN-Br was found to be 87°C.

### Example 4

One gram of the ionic liquid HO(CH₂)₃MIN-Br prepared in Synthesis Example 2 was vacuum dehydrated under heating at 110°C for 24 hours, then mixed with 10 mg of RNA and sealed in a nitrogen atmosphere. Next, while applying heat, the temperature at which the RNA dissolved was recorded. As a result, the temperature at which 10 mg of RNA completely dissolves in 1 g of the ionic liquid HO(CH₂)₃MIN-Br was found to be 82°C.

### Example 5

One gram of the ionic liquid PMIN-Cl prepared in Synthesis Example 3 was vacuum dehydrated under heating at 110°C for 24 hours, then mixed with 10 mg of DNA and sealed in a nitrogen atmosphere. Next, while applying heat, the temperature at which the DNA dissolved was recorded. As a result, the temperature at which 10 mg of DNA completely dissolves in 1 g of the ionic liquid PMIN-C1 was found to be 80° C.

### Example 6

One gram of the ionic liquid PMIN-Cl prepared in Synthesis Example 3 was vacuum dehydrated under heating at 110°C for 24 hours, then mixed with 10 mg of RNA and sealed in a nitrogen atmosphere. Next, while applying heat, the temperature at which the RNA dissolved was recorded. As a result, the temperature at which 10 mg of RNA completely dissolves in 1 g of the ionic liquid PMIN-Cl was found to be 70° C.

### Example 7

One gram of the ionic liquid PMIN-Br prepared in Synthesis Example 4 was vacuum dehydrated under heating at 110°C for 24 hours, then mixed with 10 mg of DNA and sealed in a nitrogen atmosphere. Next, while applying heat, the temperature at which the DNA dissolved was recorded. As a result, the temperature at which 10 mg of DNA completely dissolves in 1 g of the ionic liquid PMIN-Br was found to be 90° C.

### Example 8

One gram of the ionic liquid PMIN-Br prepared in Synthesis Example 4 was vacuum dehydrated under heating at 110°C for 24 hours, then mixed with 10 mg of RNA and sealed in a nitrogen atmosphere. Next, while applying heat, the temperature at which the RNA dissolved was recorded. As a result, the temperature at which 10 mg of RNA completely dissolves in 1 g of the ionic liquid PMIN-Br was found to be 82° C.

Table 1 below shows the structures of the ionic liquids used in Examples 1 to 8 above, and the temperatures at which DNA and RNA dissolve in the respective ionic liquids.

**Table 1**

| | Ionic liquid | | | | Nucleic acid dissolution temperature (°C) |
|---|---|---|---|---|---|
| | Name | Structural formula | | | |
| Example 1 | OH(CH₂)₃MIN-Cl | 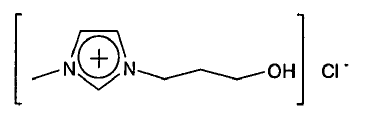 | | | 70 |
| Example 2 | | | | | 60 |
| Example 3 | OH(CH₂)₃MIN-Br | 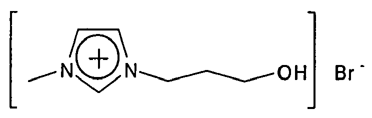 | | | 87 |
| Example 4 | | | | | 82 |
| Example 5 | PMIM-Cl | 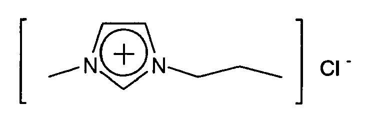 | | | 80 |
| Example 6 | | | | | 70 |
| Example 7 | PMIM-Br | 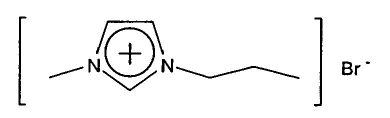 | | | 90 |
| Example 8 | | | | | 82 |

### Examples 9 and 10

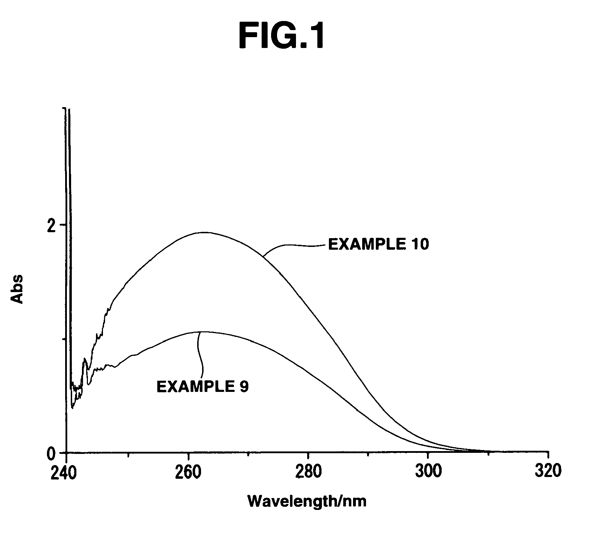

First, 2.5 g of the ionic liquid HO(CH₂)₃MIN-Cl prepared in Synthesis Example 1 was vacuum dehydrated under heating at 110°C for 24 hours. Next, 2.5 g of this ionic liquid was mixed with 1 mg (Example 9) or 1.5 mg (Example 10) of DNA, then each mixture was dissolved under heating at 84°C and subsequently left to stand at room temperature for 48 hours.
The DNA-containing solutions were each placed in square quartz cells having an optical path length of 1 mm, and their visible-ultraviolet absorption spectra were measured (UV-2500 PC spectrometer; Shimadzu Corporation) at room temperature. The resulting spectra are shown in FIG. 1.

As shown in FIG. 1, in the visible-ultraviolet absorption spectrum thus obtained, absorption near 260 nm attributable to nucleic acid bases changes with the amount of DNA addition. Also, the fact that the absorbance in the wavelength region above 320 nm where absorption by DNA does not occur was zero indicates that the DNA remains in solution within the ionic liquid even at room temperature.

### Examples 11 and 12

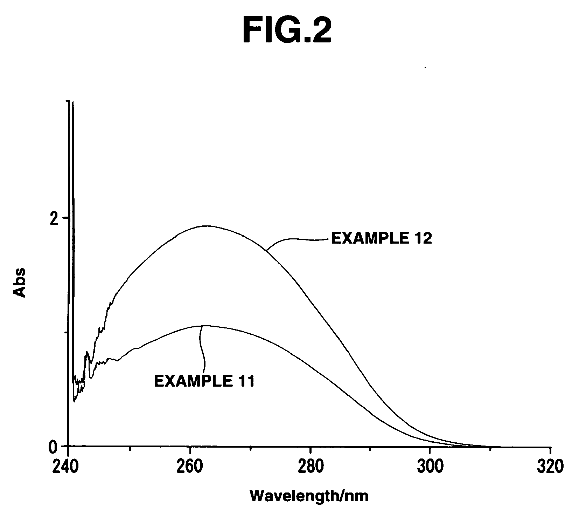

First, 2.5 g of the ionic liquid HO(CH₂)₃MIN-Cl prepared in Synthesis Example 1 was vacuum dehydrated under heating at 110°C for 24 hours. Next, 2.5 g of this ionic liquid was mixed with 1 mg (Example 11) or 1.5 mg (Example 12) of RNA, then each mixture was dissolved under heating at 74°C and subsequently left to stand at room temperature for 48 hours. The visible-ultraviolet absorption spectra of the RNA-containing solutions were measured in the same way as in Example 9. The resulting spectra are shown in FIG. 2.
As shown in FIG. 2, in the visible-ultraviolet absorption spectrum thus obtained, absorption near 260 nm attributable to nucleic acid bases changes with the amount of RNA addition. Also, the fact that the absorbance in the wavelength region above 320 nm where absorption by RNA does not occur was zero indicates that the RNA remains in solution within the ionic liquid even at room temperature.

### Example 13

The DNA-containing solution prepared in Example 1 was preserved for 120 hours, following which 3 ml of 2-propanol (Tokyo Chemical Industry) was mixed therein. The mixed solution was then centrifuged at 3,000 rpm for 15 minutes with a centrifugal separator (KA-1000; manufactured by Kubota Seisakusho KK), following which the supernatant solution was removed. Next, 3 ml of ethanol was added to the precipitate, the resulting mixture was again centrifuged at 3,000 rpm for 15 minutes, then the supernatant solution was removed.
The precipitate was dissolved in 0.2 ml of distilled water, 10 mg of sodium chloride (Wako Pure Chemical Industries) was added, following which 2 ml of ethanol was added. The resulting solution was centrifuged at 3,000 rpm for 15 minutes, and the supernatant solution was removed. The precipitate was mixed with 2 ml of an 85 wt% ethanol-water solution, and centrifuged at 3,000 rpm for 15 minutes. The supernatant solution was removed, 2 ml of an 85 wt% ethanol-water solution was again mixed with the precipitate, and the mixture was centrifuged at 3,000 rpm for 15 minutes. The supernatant solution was then removed, and the precipitate ultimately obtained was vacuum dehydrated at room temperature.

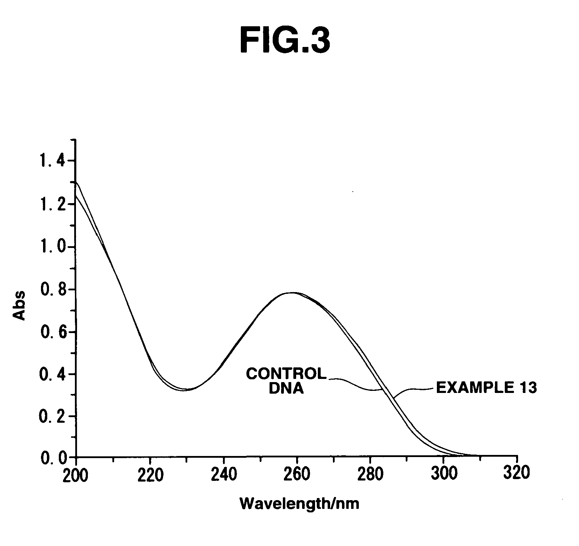

Two milligrams of the white powdered DNA obtained by drying was then dissolved in 5 ml of distilled water, and the visible-ultraviolet absorption spectrum was measured in the same way as in Example 9. The resulting spectrum is shown in FIG. 3.
As shown in FIG. 3, the spectrum shows absorption at 260 nm attributable to nucleic acid bases. The fact that an absorption spectrum similar to that of the control DNA was obtained confirmed that the white powder isolated after preservation was DNA.

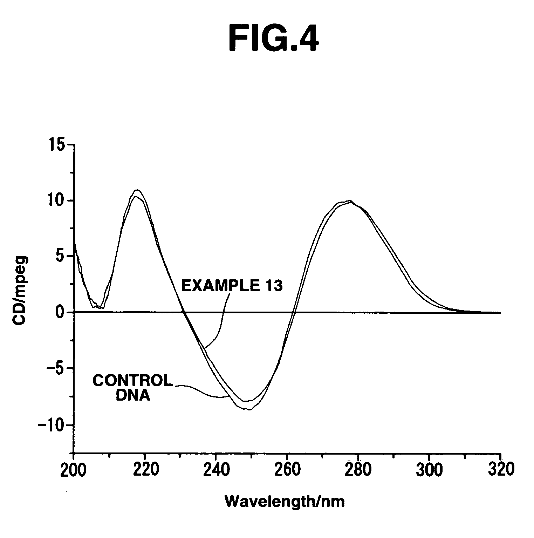

In addition, a solution obtained by dissolving 2 mg of the white powdered DNA in 5 ml of distilled water was placed in a square quartz cell having a 1 mm optical path length, and the CD spectrum was measured (J-720, manufactured by JASCO Corporation) at room temperature. The resulting spectrum is shown in FIG. 4.
As shown in FIG. 4, because a spectrum similar to that of the control DNA was obtained, the white powder isolated following preservation was confirmed to have, like natural DNA, a right-handed double chain structure like natural DNA.

### Example 14

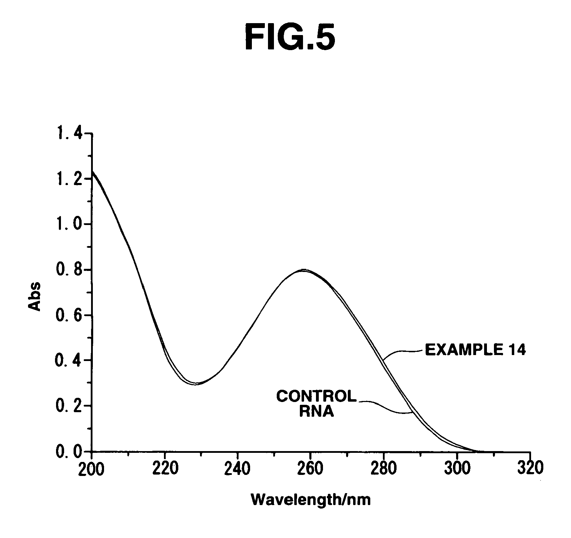

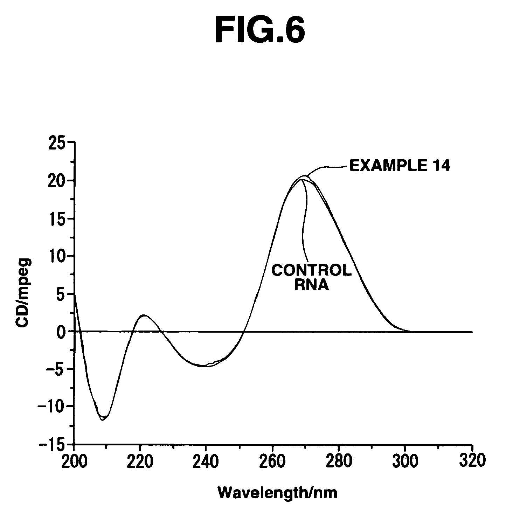

Aside from using the RNA-containing solution obtained in Example 2, white powdered RNA was isolated in the same way as in Example 13.
The visible-ultraviolet absorption spectrum and the CD spectrum of this white powdered RNA were measured in the same way as in Example 13. The resulting spectra are shown in FIGS. 5 and 6, respectively.
Referring to FIG. 5, because the absorption spectrum includes absorption at 260 nm which is attributable to nucleic acid bases and is similar to that for control RNA, the white powder isolated following preservation was confirmed to be RNA. Referring to FIG. 6, because an absorption spectrum similar to that for the control RNA was obtained, the white powder isolated following preservation was confirmed to have, like natural RNA, a right-handed double chain structure.

### Example 15

One gram of the ionic liquid EMIm-C₂COO prepared in Synthesis Example 7 was vacuum dehydrated under heating at 90°C for 36 hours, then mixed with 5 mg of DNA (Daiwa Fine Chemical) and sealed in a nitrogen atmosphere. Next, while applying heat, the temperature at which the DNA dissolved was recorded. As a result, the temperature at which 5 mg of DNA completely dissolves in 1 g of the ionic liquid EMIm-C₂COO was found to be 70°C.

### Example 16

One gram of the ionic liquid EMIm-C₂COO prepared in Synthesis Example 7 was vacuum dehydrated under heating at 90°C for 36 hours, then mixed with 5 mg of RNA (Tokyo Kasei Kogyo) and sealed in a nitrogen atmosphere. Next, while applying heat, the temperature at which the RNA dissolved was recorded. As a result, the temperature at which 5 mg of RNA completely dissolves in 1 g of the ionic liquid EMIm-C₂COO was found to be 60°C.

### Example 17

One gram of the ionic liquid EMIm-HCOO prepared in Synthesis Example 8 was vacuum dehydrated under heating at 90°C for 36 hours, then mixed with 5 mg of DNA and sealed in a nitrogen atmosphere. Next, while applying heat, the temperature at which the DNA dissolved was recorded. As a result, the temperature at which 5 mg of DNA completely dissolves in 1 g of the ionic liquid EMIm-HCOO was found to be 60°C.

### Example 18

One gram of the ionic liquid EMIm-HCOO prepared in Synthesis Example 8 was vacuum dehydrated under heating at 90°C for 36 hours, then mixed with 5 mg of RNA and sealed in a nitrogen atmosphere. Next, while applying heat, the temperature at which the RNA dissolved was recorded. As a result, the temperature at which 5 mg of RNA completely dissolves in 1 g of the ionic liquid EMIm-HCOO was found to be 48°C.
Table 2 below shows the structures of the ionic liquids used in Examples 15 to 18 above, and the temperatures at which DNA and RNA dissolve in the respective ionic liquids.

**Table 2**

| | Ionic liquid | | | | Nucleic acid dissolution temperature (° C) |
|---|---|---|---|---|---|
| | Name | Structural formula | | | |
| Example 15 | EMIm-C₂COO | 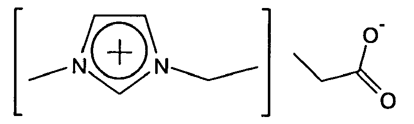 | | | 70 |
| Example 16 | | | | | 60 |
| Example 17 | EMIm-HCOO | 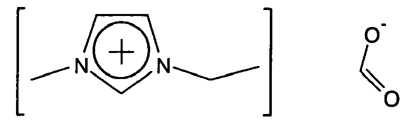 | | | 60 |
| Example 18 | | | | | 48 |

### Example 19

One gram of the ionic liquid MIm-Cl prepared in Synthesis Example 9 was vacuum dehydrated under heating at 70°C for 16 hours, then mixed with 10 mg of DNA (Nichiro Corporation) and sealed in a nitrogen atmosphere. Next, while applying heat, the temperature at which the DNA dissolved was recorded. As a result, the temperature at which 10 mg of DNA completely dissolves in 1 g of the ionic liquid MIm-Cl was found to be 76°C.

### Example 20

One gram of the ionic liquid MIm-HCOO prepared in Synthesis Example 10 was vacuum dehydrated under heating at 70°C for 16 hours, then mixed with 10 mg of DNA (Nichiro Corporation) and sealed in a nitrogen atmosphere. Next, while applying heat, the temperature at which the DNA dissolved was recorded. As a result, the temperature at which 10 mg of DNA completely dissolves in 1 g of the ionic liquid MIm-HCOO was found to be 85°C.

Table 3 below shows the structures of the ionic liquids used in Examples 19 and 20 above, and the temperatures at which DNA dissolve in the respective ionic liquids.

**Table 3**

| | Ionic liquid | | | | Nucleic acid dissolution temperature (°C) |
|---|---|---|---|---|---|
| | Name | Structural formula | | | |
| Example 19 | MIm-Cl | 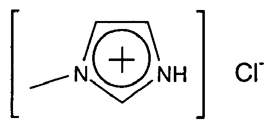 | | | 76 |
| Example 20 | MIm-HCOO | 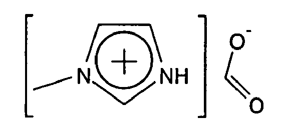 | | | 85 |

## Claims

1. A solvent for dissolving nucleic acid, **characterized by** comprising an ionic liquid which can dissolve nucleic acid.

2. The solvent for dissolving nucleic acid of claim 1 which is **characterized in that** the ionic liquid is composed of at least one cation selected from among ammonium cations, imidazolium cations and pyridinium cations.

3. The solvent for dissolving nucleic acid of claim 1 or 2 which is **characterized in that** the ionic liquid is composed of an anion which is a halide ion or a carboxylic acid ion having a total of 1 to 3 carbons.

4. The solvent for dissolving nucleic acid of claim 1 which is **characterized in that** the ionic liquid is a neutralized ionic liquid.

5. The solvent for dissolving nucleic acid of any one of claims 1 to 4 which is **characterized by** being adapted to preserve nucleic acid or to react nucleic acid.

6. A nucleic acid-containing solution, **characterized by** comprising nucleic acid dissolved in an ionic liquid.

7. A method for preserving nucleic acid, **characterized by** preserving nucleic acid in a dissolved state within an ionic liquid.
